# EUROPEAN PATENT APPLICATION

(11) **EP 1 690 532 A1**
(43) Date of publication of application: **16.08.2006**
(21) Application number: 05002933.9
(22) Date of filing: 11.02.2005
(51) Int. Cl.: A61K 9/51, A61K 33/00, A61K 9/16

(54) **Device for gastric treatment and manufacturing process for the same**

(71) Applicant: NOLabs AB, 252 21 Helsingborg (SE)
(72) Inventor: Peters, Tor, 8703 Erlenbach (CH)
(74) Representative: Karlsson, Leif Gunnar Börje

(57) **Abstract**

A device is provided that allows for target treatment of gastric and gastrointestinal complications, including gastric ulcer. The device comprises a nitric oxide (NO) eluting polymer arranged to contact the area to be treated, such that a therapeutic dose of nitric oxide is eluted from said nitric oxide eluting polymer to said area. Furthermore, a manufacturing method for said device is disclosed.

## Description

### Field of the Invention

This invention pertains in general to the field of treatment of gastric and gastrointestinal complications, such as gastric ulcer. More particularly the invention relates to a device for gastric and gastrointestinal treatment, and a process for manufacturing of said device, involving the use of nitric oxide (NO).

### Background of the Invention

About 10 percent of the population experience some kind of gastric and gastrointestinal complications, such as gastric ulcer, during some point of time during their life. Some of these persons experience long lasting problems, and their gastric and gastrointestinal complications may be looked upon as chronic. The hectic way life style of the western world is major factor in respect of gastric and gastrointestinal complications, such as gastric ulcer.

For years it is the understanding that stress, smoking, coffee drinking, consumption of alcohol, bad diet etc, are the main causes of gastric ulcers. In fact, gastric and gastrointestinal complications, such as gastric ulcer, are developed as a result of unbalance between stomach acid and pepsin and the defence mechanism of the mucous membrane in the stomach against these corroding substances. The last couple of years it has been established that gastric and gastrointestinal complications, such as gastric ulcer, develop during simultaneous presence of *Helicobacter Pylori* in the stomach. When *Helicobacter Pylori* is present in the oesophagus it is not pathogenic. *Helicobacter Pylori* has only a pathogenic effect in the stomach.

The treatment of gastric and gastrointestinal complications, such as gastric ulcers, is a huge market, and a lot different medications relating to this matter have been widely discussed, others have been very lucrative. These medications concentrate on controlling the production of stomach acid, and the unbalance between stomach acid and pepsin and the defence mechanism of the mucous membrane. In respect of the infection of *Helicobacter Pylori* the only treatment is antibiotics. Treatment with antibiotics has certain disadvantages, such as that the bacteria develops tolerance and resistance to the antibiotics over time, and thus become difficult to eradicate.

It is known that nitric oxide (NO) provides an alternative to conventional therapies, such as antibiotics. Nitric oxide is a highly reactive molecule that is involved in many cell functions. In fact, nitric oxide plays a crucial role in the immune system and is utilized as an effector molecule by macrophages to protect itself against a number of pathogens, such as fungi, viruses, bacteria etc., and general microbial invasion. This improvement of healing is partly caused by NO inhibiting the activation or aggregation of blood platelets, and also by NO causing a reduction of inflammatory processes at the site of an implant.

NO is also known to have an anti-pathogenic, especially an anti-viral, effect, and furthermore NO has an anti-cancerous effect, as it is cytotoxic and cytostatic in therapeutic concentrations, i.e. it has among other effects tumoricidal and bacteriocidal effects. NO has for instance cytotoxic effects on human haematological malignant cells from patients with leukaemia or lymphoma, whereby NO may be used as a chemotherapeutic agent for treating such haematological disorders, even when the cells have become resistant to conventional anti-cancer drugs. This anti-pathogenic and anti-tumour effect of NO is taken advantage of by the present invention, without having adverse effects as for instance many anti-cancer drugs.

However, due to the short half-life of NO, it has hitherto been very hard to treat viral, bacteria, virus, fungi or yeast infections with NO. This is because NO is actually toxic in high concentrations and has negative effects when applied in too large amounts to the body. NO is actually also a vasodilator, and too large amounts of NO introduced into the body will cause a complete collapse of the circulatory system. On the other hand, NO has a very short half-life of fractions of a second up to a few seconds, once it is released. Hence, administration limitations due to short half-life and toxicity of NO have been limiting factors in the use of NO in the field of anti-pathogenic and anti-cancerous treatment so far.

In recent years research has been directed to polymers with the capability of releasing nitrogen oxide when getting in contact with water. Such polymers are for example polyalkyleneimines, such as L-PEI (Linear PolyEthyleneImine) and B-PEI (Branched PolyEthyleneImine), which polymers have the advantage of being biocompatible, after the release of nitrogen oxide.

Other example for NO eluting polymers are given in US-5,770,645, wherein polymers derivatized with at least one -NOₓ group per 1200 atomic mass unit of the polymer are disclosed, X being one or two. One example is an S-nitrosylated polymer and is prepared by reacting a polythiolated polymer with a nitrosylating agent under conditions suitable for nitrosylating free thiol groups.

Akron University has developed NO-eluting L-PEI molecule that can be nano-spun onto the surface of medical devices to be permanently implanted in the body, such as implanted grafts, showing significant improvement of the healing process and reduced inflammation when implanting such devices. According to US-6,737,447, a coating for medical devices provides nitric oxide delivery using nanofibers of linear poly(ethylenimine)-diazeniumdiolate. Linear poly(ethylenimine)diazeniumdiolate releases nitric oxide (NO) in a controlled manner to tissues and organs to aid the healing process and to prevent injury to tissues at risk of injury. Electrospun nano-fibers of linear poly(ethylenimine) diazeniumdiolate deliver therapeutic levels of NO to the tissues surrounding a medical device while minimizing the alteration of the properties of the device. A nanofiber coating, because of the small size and large surface area per unit mass of the nanofibers, provides a much larger surface area per unit mass while minimizing changes in other properties of the device.

However, the disclosure is both silent concerning an improvement of present technology in respect of treatment of gastric and gastrointestinal complications, such as gastric ulcer, and the anti pathogenic potential of nitric oxide.

Hence, an improved device for the treatment and/or prevention of gastric and gastrointestinal complications, such as gastric ulcers, which device presents the possibility to treat and/or prevent both ulcers and the occurrence of *Helicobacter Pylori* in the same time, does not develop resistance against the active pharmaceutical substance, is easy to apply, provides improved circulation in form of a vasodilating effect, provides a painless treatment, has fast inset of treatment effect, would be advantageous, and in particular a device allowing for target prevention and treatment of gastric and gastrointestinal complications, such as gastric ulcer, would be advantageous.

### Summary of the Invention

Accordingly, the present invention preferably seeks to mitigate, alleviate or eliminate one or more of the above-identified deficiencies in the art and disadvantages singly or in any combination and solves at least the problems mentioned above, by providing a device according to the appended patent claims.

According to one aspect of the invention, a device is provided that allows for target treatment of gastric and gastrointestinal complications, such as gastric ulcer. The device comprises a nitric oxide (NO) eluting polymer arranged to contact the area to be treated, such that a therapeutic dose of nitric oxide is eluted from said nitric oxide eluting polymer to said area.

According to another aspect of the invention, a manufacturing process for such a device is provided, wherein the process is a process for forming a device that allows for target treatment of gastric and gastrointestinal complications, such as gastric ulcer. The process comprises selecting a plurality of nitric oxide eluting polymeric particles, such as nano fibres, fibres, nano particles, or microspeheres, and deploying said nitric oxide eluting particles into forms such as nano-particles, micro-spheres, or powder to be comprised in said device.

The present invention has at least the advantage over the prior art that it presents the possibility to treat and/or prevent both ulcers and the occurrence of *Helicobacter Pylori* in the same time, does not develop resistance against the active pharmaceutical substance, is easy to apply, provides improved circulation in form of a vasodilating effect, provides a painless treatment, and has fast inset of treatment effect, by the exposure of an infected and/or wounded area to NO, whereby a very effective anti-gastric and anti-gastrointestinal ulcer therapy is achievable.

### Brief Description of the Drawings

These and other aspects, features and advantages of which the invention is capable of will be apparent and elucidated from the following description of embodiments of the present invention, reference being made to the accompanying drawings, in which
Fig. 1 is a schematic illustration of a nano-particles, or micro-spheres, according to the invention,
Fig. 2 is a schematic illustration of a drink according to the invention, and
Fig. 3 is a schematic illustration of a capsule/pill according to the invention.

### Description of Embodiments

The following description focuses on embodiments of the present invention applicable to a device, in form of nano-particles, or micro-spheres, which allows for target treatment of gastric and gastrointestinal complications, such as gastric ulcer or intestinal cancer, such as colon cancer.

With regard to nitric oxide (nitrogen monoxide, NO), its physiological and pharmacological roles have attracted much attention and thus have been studied. NO is synthesized from arginine as the substrate by nitric oxide synthase (NOS). NOS is classified into a constitutive enzyme, cNOS, which is present even in the normal state of a living body and an inducible enzyme, iNOS, which is produced in a large amount in response to a certain stimulus. It is known that, as compared with the concentration of NO produced by cNOS, the concentration of NO produced by iNOS is 2 to 3 orders higher, and that iNOS produces an extremely large amount of NO.

In the case of the generation of a large amount of NO as in the case of the production by iNOS, it is known that NO reacts with active oxygen to attack exogenous microorganisms and cancer cells, but also to cause inflammation and tissue injury. On the other hand, in the case of the generation of a small amount of NO as in the case of the production by cNOS, it is considered that NO takes charge of various protective actions for a living body through cyclic GMP (cGMP), such as vasodilator action, improvement of the blood circulation, antiplatelet-aggregating action, antibacterial action, anticancer action, acceleration of the absorption at the digestive tract, renal function regulation, neurotransmitting action, erection (reproduction), learning, appetite, and the like. Heretofore, inhibitors of the enzymatic activity of NOS have been examined for the purpose of preventing inflammation and tissue injury, which are considered to be attributable to NO generated in a large amount in a living body. However, the promotion of the enzymatic activity (or expressed amount) of NOS (in particular, cNOS) has not been examined for the purpose of exhibiting various protective actions for a living body by promoting the enzymatic activity of NOS and producing NO appropriately.

In recent years research has been directed to polymers with the capability of releasing nitrogen oxide when getting in contact with water. Such polymers are for example polyalkyleneimines, such as L-PEI (Linear PolyEthyleneImine) and B-PEI (Branched PolyEthyleneImine), which polymers have the advantage of being biocompatible, after the release of nitrogen oxide.

The polymers according to the present invention may be manufactured by electro spinning. Electro spinning is a process by which a suspended polymer is charged. At a characteristic voltage a fine jet of polymer releases from the surface in response to the tensile forces generated by interaction by an applied electric field with the electrical charge carried by the jet. This process produces a bundle of polymer fibres, such as nano-fibres. This jet of polymer fibres may be directed to a surface to be treated.

Furthermore, US 6,382,526, US 6,520,425, and US 6,695,992 disclose processes and apparatuses for the production of such polymeric fibres. These techniques are generally based on gas stream spinning, also known within the fiber forming industry as air spinning, of liquids and/or solutions capable of forming fibers.

Other example for NO eluting polymers are given in US-5,770,645, wherein polymers derivatized with at least one -NOX group per 1200 atomic mass unit of the polymer are disclosed, X being one or two. One example is an S-nitrosylated polymer and is prepared by reacting a polythiolated polymer with a nitrosylating agent under conditions suitable for nitrosylating free thiol groups.

Akron University has developed NO-eluting L-PEI molecule that can be nano-spun onto the surface of medical devices such as implanted grafts, showing significant improvement of the healing process and reduced inflammation when implanting such devices. According to US-6,737,447, a coating for permanently implanted medical devices provides nitric oxide delivery using nanofibers of linear poly(ethylenimine)-diazeniumdiolate. Linear poly(ethylenimine)diazeniumdiolate releases nitric oxide (NO) in a controlled manner. Another advantage of L-PEI is that NO is released without any secondary products that could lead to undesired side effects.

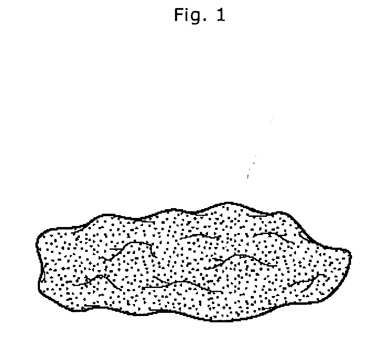

In an embodiment of the invention, according to Fig. 1, the device is embodied in form of nano-particles, or micro spheres. These nano-particles, or micro-spheres, may be formed from the NO-eluting polymers according to the present invention. These nano-particles, or micro-spheres, may be swallowed for accessing the gastro-intestinal tract of a body. When the nano-particles, or micro-spheres, get in contact with the moisture in the stomach, the NO-eluting polymer starts to elute NO on the area to be treated, such as the stomach. At this point an effective healing process of the ulcer is initiated at the same time as an antimicrobial effect against the *Helicobacter Pylori* sets in.

In another embodiment the nano-particles, or micro-spheres, according to the present invention may be formed from the NO-eluting polymers according to the present invention, encapsulated, or integrated, in any suitable material, such as polyvinylacetates, polylacticacids, starch, cellulose, polyhydroxyalkanoates, polycaprolactone, polyvinylalcohol, protein based plastics, gelatine, biogradable or biocompatible polymers, and other soluble plastics. The integration of, or encapsulation in, these materials is performed to regulate and/or control the elution of NO in the stomach, and to provide continuous exposure of the gastrointestinal tract to NO. The encapsulation may be such that the material breaks by the movement of the stomach, or dissolves in the aqueous acidic environment in the stomach.

In yet another embodiment of the present invention, the device is in form of a powder, said powder being obtained by grounding or pulverizing the NO-eluting polymer according to the invention.

When the nano-particles, or micro-spheres, or powder, according to this embodiment, gets in contact with the moisture in the stomach, they/it start/starts to elute NO on the area to be treated. At this point an effective healing process of the ulcer is initiated at the same time as an antimicrobial effect against the *Helicobacter Pylori* sets in.

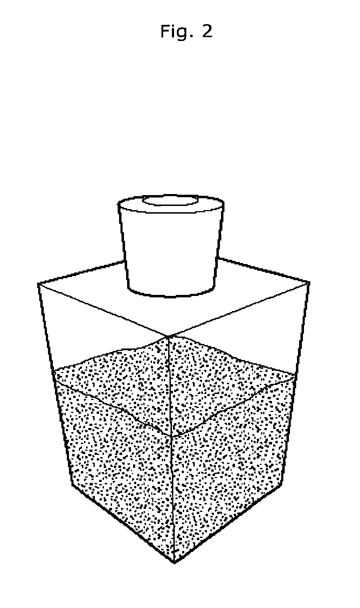

In still another embodiment the nano-particles, or micro-spheres, are encapsulated in a suitable, acid dissolving, but not aqueous dissolving, material and integrated in a suitable liquid, said liquid being drinkable, according to Fig. 2. When the liquid reaches the stomach the encapsulation material is dissolved and the nano-particles, or micro-spheres starts to elute NO. At this point an effective healing process of the ulcer is initiated at the same time as an antimicrobial effect against the *Helicobacter Pylori* sets in.

In another embodiment of the present invention the nano-particles, or micro-spheres, or ground/pulverized NO-eluting polymer, may be encapsulated in a suitable material, such as gelatine, starch, cellulose etc, to be swallowed. When the thus obtained capsule reaches the stomach, the gelatine dissolves and the nano-particles, or micro-spheres, starts to elute NO.

In still another embodiment of the present invention the nano-particles, or micro-spheres, or ground/pulverized NO-eluting polymer may be encapsulated in a material that is insoluble in the acidic environment of the stomach. In this embodiment the capsule is instead dissolved in the basic environment of the duodenum, to treat gastric and gastrointestinal complications in this region.

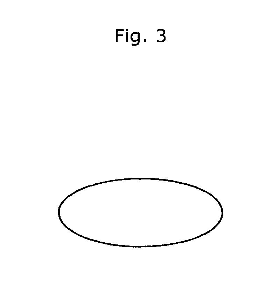

In another embodiment of the present invention the nano-particles, or micro-spheres, or ground/pulverized NO-eluting polymer are/is compressed into a pill, tablet or pellet, which pill, tablet or pellet, according to Fig. 3, then is swallowed. When the pill, tablet, or pellet, reaches the stomach, an effective healing process of the ulcer is initiated at the same time as an antimicrobial effect against the *Helicobacter Pylori* sets in.

In yet another embodiment of the present invention the NO-eluting device may be combined with, or acting as a booster for, pharmaceuticals, vitamins, nicotin, nitroglycerin etc. This embodiment presents a device with the advantage of combining two therapeutic treatments, of significant value, in one treatment. A specific example of this embodiment is a combination of the device according to the present invention and active substances in respect of gastric ulcer. Hence, a synergetic effect may be achieved by such devices when NO that is eluted from the device. NO has for instance a vasodilatory effect on the region where the device having the combination compound actuates. Vasodilated tissue is more susceptible to certain medications and thus more easily treated by the medical preparations and still NO has in addition to that the anti-inflamatory, anti-bacterial etc. effect. Hence, an unexpected surprisingly effective treatment is provided.

In another embodiment of the device according to the present invention, the nano-particles, or micro-spheres, are integrated in a gel. It may also be integrated in a hydrogel, which is mixed directly before use. This gel is then swallowed, and the device elutes NO when the gel, or hydrogel, reaches the stomach. This embodiment has the advantage of being able to penetrate pockets and corners in the gastrointestinal tract for closer elution of NO on the area to be treated.

In another embodiment of the present invention the nano-particles, or micro-spheres, of the polymers in the present invention, may be encapsulated in a material that breaks upon the stress from chewing. Then said nano-particles, or micro-spheres, may be integrated in chewing gum. This kind of chewing gum may then be used to prevent or treat gastric and gastrointestinal complications, such as gastric ulcer. The materials used to encapsulate these nano-particles, or micro-spheres, may be chosen from the group comprising polyvinylacetates, polylacticacids, starch, cellulose, polyhydroxyalkanoates, polyesters, polycaprolactone, polyvinylalcohol, protein based plastics, gelatine, biogradable polymers, and other soluble plastics. When the chewing gum is chewed the nano-particles, or micro-spheres, of NO-eluting polymer are swallowed, and starts to elute NO in the stomach or gastrointestinal tract. The encapsulation materials provide the possibility to regulate and control the elution of NO. This embodiment has the advantages that it provides another option of a way to obtain a continuous dosage of NO is easy to apply, the treatment effect covers the whole oral cavity, and it is easy to manufacture.

In another embodiment the nano-particles, or micro-spheres, of the polymers in the present invention, may be integrated in a film. When the film is put in the mouth the nano-particles, or micro-spheres, of NO-eluting polymer elutes NO that is swallowed, and said NO treats complications in the stomach or gastrointestinal tract. The encapsulation materials, according to above, provides the possibility to regulate and control the elution of NO. The film may also be swallowed before dissolving, thus effecting directly the stomach/intestinal tract.

In yet another embodiment of the present invention the device is in form of a foam or cream.

When the NO-eluting device according to the present invention gets in contact with the moisture in the stomach, the NO-eluting device starts to release NO to the area to be treated. This device does not develop resistance against nitric oxide (NO), is easy to apply, provides a painless treatment, has fast inset of treatment effect, and combines an ulcer treatment effect with an antimicrobial effect, such as an *anti-Helicobacter Pylori* effect.

In another embodiment of the device according to the present invention, it is manufactured of a basic carrier material, which basic material is integrated, or covered, with the nano-fibres, nano-particles, powder and/or micro-spheres of NO-eluting polymer according to the invention. When the basic material, for example in form of small granules, reaches the stomach, the nano-fibres, nano-particles, powder and/or micro-spheres of NO-eluting polymer start to elute NO on the area to be treated. At this point an effective healing process of the ulcer is initiated at the same time as an antimicrobial effect against the *Helicobacter Pylori* sets in. The granules, according to this embodiment of the invention, then passes through the gastrointestinal tract, and exit the body in the faeces.

The basic material of this device, according to the invention, may be polyesters, polyamides, polyethers, polyurethanes, polycarbonates, polyvinylacetates, polylacticacids, starch, cellulose, polyhydroxyalkanoates, polypropylene, cotton, polyesters, polycaprolactone, polyvinylalcohol, polyacrylonitrile, polystyrene, poly(acrylic acid), polypropylene, protein based plastics, gelatine, and other biocompatible polymers. The NO-eluting polymer, such as L-PEI, may be integrated in, spun together with, or spun on top of, any of these materials.

When placed on an area to be treated the device according to the present invention provides prevention and treatment of gastric and gastrointestinal complications, such as gastric ulcer or in certain cases for instance intestinal cancer.

The device according to the present invention elutes nitric oxide (NO) from said eluting polymer in a therapeutic dose in the stomach, or dudenum, such as between 1 to 100 ppm, such as 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90 91, 92, 93, 94, 95, 96, 97, 98, 99, or 100 ppm.

The NO-eluting polymers in the devices according to the present invention may be combined with silver, such as hydroactivated silver. The integration of silver in the devices according to the present invention gives the healing process an extra boost. Preferably the silver is releasable from the devices in the form of silver ions.

In the embodiments of the present invention it may be suitable to control or regulate the time span of NO release from the device according to the invention. This may be accomplished by integrating other polymers or materials in said device. These polymers or materials may be chosen from any suitable material or polymer, such as polyvinylacetates, polylacticacids, starch, cellulose, polyhydroxyalkanoates, polyesters, polycaprolactone, polyvinylalcohol, protein based plastics, gelatine, biogradable polymers, and other soluble plastics.

The device according to the present invention may be manufactured by, for example electro spinning of L-PEI or other polymers comprising L-PEI or being arranged in combination with L-PEI. L-PEI is the charged at a characteristic voltage, and a fine jet of L-PEI releases as a bundle of L-PEI polymer fibres. This jet of polymer fibres may be directed to a surface to be treated. The surface to be treated may for example be any suitable material in respect of a device according to the present invention. The electro spun fibres of L-PEI then attach on said material and form a coating/layer of L-PEI on the device according to the invention. Such fibres are also easily further processed to other forms, such as the above mentioned powder, which simply is obtainable by applying a shredder or blender type apparatus to the fibres until a powder of desired granulation size is received.

It is of course possible to electro spin the other NO-eluting polymers, according to above, on the device according to the invention while still being inside the scope of the present invention.

In one embodiment the NO-eluting polymers according to the present invention are electro spun in such way that pure NO-eluting polymer fibres may be obtained.

Gas stream spinning, or air spinning, of said NO-eluting polymers onto the device according to the present invention is also within the scope of an embodiment of the manufacturing method according to the present invention.

The manufacturing process according to the present invention presents the advantages of providing devices with large contact surface of the NO-eluting polymer fibres and with the area to be treated, effective use of NO-eluting polymer, and a cost effective way of producing the device according to the present invention.

Hereinafter, some potential uses of the present invention are described:
A method of therapeutical treatment of gastric and gastrointestinal complications, including gastric ulcer by means of a device comprises a nitric oxide (NO) eluting polymer configured for eluting a therapeutic dosage of nitrogen oxide (NO) when used for said treatment, comprising exposing said treatment site of said complication in or on a body to said nitric oxide when said polymer in use elutes nitrogen oxide (NO) by eluting a therapeutic dose of nitric oxide from said nitric oxide eluting polymer to said treatment site.
The method according to the above, wherein said site of said complication is the stomach, or the gastrointestinal tract, and wherein said method comprises applying nano-particles or micro-spheres, a pill, a tablet, a pellet, a drink, a gel, a hydrogel, a foam, a cream, granules, and/or a capsule to said site for said exposure.
Use of nitric oxide (NO) in a therapeutic dose for therapeutically treating gastric ulcer and/or intestinal cancer.
The invention may be implemented in any suitable form. The elements and components of the embodiments according to the invention may be physically, functionally, and logically implemented in any suitable way. Indeed, the functionality may be implemented in a single unit, in a plurality of units, or as part of other functional units.
Although the present invention has been described above with reference to specific embodiments, it is not intended to be limited to the specific form set forth herein. Rather, the invention is limited only by the accompanying claims and, other embodiments than the specific above are equally possible within the scope of these appended claims.
In the claims, the term "comprises/comprising" does not exclude the presence of other elements or steps. Furthermore, although individually listed, a plurality of means, elements or method steps may be implemented. Additionally, although individual features may be included in different claims, these may possibly advantageously be combined, and the inclusion in different claims does not imply that a combination of features is not feasible and/or advantageous. In addition, singular references do not exclude a plurality. The terms "a", "an", "first", "second" etc do not preclude a plurality. Reference signs in the claims are provided merely as a clarifying example and shall not be construed as limiting the scope of the claims in any way.

## Claims

1. A device configured for therapeutic target treatment and/or prevention of gastric and gastrointestinal complications, including gastric ulcer, wherein
said device comprises a nitric oxide (NO) eluting polymer configured for eluting a therapeutic dosage of nitrogen oxide (NO)configured for exposing an area of treatment in the stomach or gastrointestinal tract to said nitric oxide when said polymer in use elutes nitrogen oxide (NO).

2. Device according to claim 1, wherein said polymer is selected from the group comprising polyalkyleneimines, S-nitrosylated polymer, and poly(alkylenimine)diazeniumdiolates, or any combinations thereof.

3. Device according to claim 1, wherein said polymer is L-PEI (linear polyethyleneimine), loaded with nitric oxide(NO), arranged for release of the nitric oxide (NO) at said stomach or gastrointestinal tract.

4. Device according to claim 1, in a form selected from the group consisting of powder, nano-particles or micro-spheres, pill, tablet, pellet, drink, gel, hydrogel, foam, cream, granules, chewing gum, film, and capsule, or combinations thereof.

5. Device according to claim 1, wherein said device is partly disintegrable when subjected to moisture or water.

6. Device according to claim 1, wherein said polymer comprises silver, configured for said therapeutic target treatment and/or prevention of gastric and gastrointestinal complications of the stomach or gastrointestinal tract.

7. Device according to claim 1, wherein said polymer is acting as a booster for pharmaceuticals, vitamins, and drugs, or combinations thereof, combined with silver.

8. Device according to claim 4, wherein said nano-particles, or micro-spheres, are encapsulated, or integrated, in a material, selected from the group consisting of polyvinylacetates, polylacticacids, starch, cellulose, polyhydroxyalkanoates, polyesters, polycaprolactone, polyvinylalcohol, protein based plastics, and/or gelatine, and combinations thereof, for regulating and/or controlling elution of NO.

9. Device according to claim 8, wherein said nano-particles, or micro-spheres, are integrated in a drink, gel, hydrogel, chewing gum, film, or foam, and combinations thereof.

10. Device according to claim 5, wherein said granules are of a material, selected from the group consisting of polyesters, polyamides, polyethers, polyurethanes, polycarbonates, polyvinylacetates, polylacticacids, starch, cellulose, polyhydroxyalkanoates, polypropylene, cotton, polyesters, polycaprolactone, polyvinylalcohol, polyacrylonitrile, polystyrene, poly(acrylic acid), polypropylene, protein based plastics, gelatine, and other biocompatible polymers, and combinations thereof, integrated, or covered, with said NO-eluting polymer.

11. A manufacturing process for a device according to claim 1, comprising
selecting a plurality of nitric oxide eluting polymeric particles, preferably nano fibres, nano particles or micro spheres, and
deploying said nitric oxide eluting polymeric particles into a suitable form, , or as a coating onto a carrier, to form said device,
said deploying comprises electro, air, or gas stream spinning of said particles.

12. Use of a nitric oxide (NO) eluting polymer for the manufacture of a device for the treatment of gastric and gastrointestinal complications, including gastric ulcer wherein
nitric oxide is loaded to said device so that said device elutes nitric oxide (NO) from said eluting polymer in a therapeutic dose when used.

13. Use according to claim 12, wherein said therapeutic dose is 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90 91, 92, 93, 94, 95, 96, 97, 98, 99, or 100 ppm.
